# EUROPEAN PATENT SPECIFICATION

(11) **EP 3 885 355 B1**
(45) Date of publication and mention of the grant of the patent: **28.10.2026**
(21) Application number: 20165419.1
(22) Date of filing: 24.03.2020
(51) Int. Cl.: C07K 14/46, C12N 15/82

(54) **A VIRAL MOLECULE FOR PROTECTING PLANTS AGAINST THE BACTERIA XYLELLA FASTIDIOSA**
EIN VIRALES MOLEKÜL ZUM SCHUTZ VON PFLANZEN GEGEN DIE BACTERIA XYLELLA FASTIDIOSA
MOLÉCULE VIRALE POUR LA PROTECTION DES PLANTES CONTRE LES BACTÉRIES XYLELLA FASTIDIOSA

(43) Date of publication of application: 29.09.2021
(73) Proprietor: Universidade de Évora, 7000-803 Évora (PT)
(72) Inventor: Materatski, Patrick, 7005-591 Évora (PT); Campos, Maria, 7005-593 Évora (PT); Varanda, Carla, 7005-591 Évora (PT); Félix, Maria, 7005-401 Évora (PT); Peixe, Augusto, 7000-757 Évora (PT); Basaloco Fonseca, Margarida, Estremoz (PT)
(74) Representative: Pereira da Cruz, Joao

(56) References cited:
- EP-A2- 0 552 559
- L.V. KUZINA ET AL: "In vitro activities of antibiotics and antimicrobial peptides against the plant pathogenic bacterium Xylella fastidiosa", LETTERS IN APPLIED MICROBIOLOGY, vol. 42, no. 5, 1 May 2006 (2006-05-01), GB, pages 514 - 520, XP055731917, ISSN: 0266-8254, DOI: 10.1111/j.1472-765X.2006.01898.x
- CARDOSO J M S ET AL: "First characterization of infectious cDNA clones of olive mild mosaic virus", PHYTOPATHOLOGIA MEDITERRANEA,, vol. 51, no. 2, 1 January 2012 (2012-01-01), pages 259 - 265, XP002800369
- "Olive mild mosaic virus isolate GP clone pUFLOMMV-5, complete genome", EMBL,, 2 December 2011 (2011-12-02), XP002800370
- CARLA M. R. VARANDA ET AL: "Genetic Diversity of the Coat Protein of Olive Mild Mosaic Virus (OMMV) and Tobacco Necrosis Virus D (TNV-D) Isolates and Its Structural Implications", PLOS ONE, vol. 9, no. 10, 28 October 2014 (2014-10-28), pages e110941, XP055730622, DOI: 10.1371/journal.pone.0110941
- XIAOQING SHI ET AL: "Efficient production of antifungal proteins in plants using a new transient expression vector derived from tobacco mosaic virus", PLANT BIOTECHNOLOGY JOURNAL, vol. 17, no. 6, 6 June 2019 (2019-06-06), GB, pages 1069 - 1080, XP055731335, ISSN: 1467-7644, DOI: 10.1111/pbi.13038

## Description

### Technical domain of the invention

The present invention relates to a viral molecule comprising a virus-based vector that is able to confer protection of plants against *Xylella fastidiosa,* the pathogen that causes the Quick Decline Syndrome in olive. In another aspect, the invention relates to a method for controlling said bacteria in plants by applying a phytosanitary composition comprising a virus-based vector, carrying a peptide sequence that provides plant protection against *Xylella fastidiosa.*

Therefore, the present invention is in the technical filed of plant disease control, microbiology and biotechnology.

### Background of the invention

*Xylella fastidiosa* is a Gram-negative xylem limited, vector transmitted, and a quarantine pathogen listed as A1 by the European and Mediterranean Plant Protection Organization (EPPO). This bacterium is known to cause important economically diseases, including Pierce's disease of grapevine, leaf scorch of almond, oleander and coffee, citrus variegation chlorosis, and others.

It was found in Salento Peninsula, southern Italy, associated to olive trees that began to decline and die, a condition of unknown aetiology called 'Olive quick decline syndrome' whose symptoms include withering and desiccation of scattered shots and small branches that extend to the entire canopy, followed by a collapse of the trees. The affected area was of about 10,000 ha, between 2010 and 2013 and all symptomatic trees revealed to be positive for *X. fastidiosa.* This was the first widespread detection of the bacterium in Europe and quarantine measures were immediately imposed by the European Food Safety Authority (EFSA).

X. *fastidiosa* is transmitted by xylem fluid feeding leafhoppers (especially members of the superfamily Cercopoidea), in particular by *Philaenus spumarius* (Hemiptera: Aphrophoridae) as a possible vector of *X. fastidiosa* in olive, in Italy. However, any xylem fluid feeding hemipteran should be regarded as a potential vector of this bacterium.

Document WO2016185380A1 describes the use of a hydroxyapatite as a carrier of bioactive substances comprising a metal ion for the treatment of vascular diseases.

Document WO2018234690A1 discloses plant extract compositions for stimulating plant defenses against plant diseases including the ones cause by *X. fastidiosa.*

Document WO2014063070A2 describes compositions based on a bacteriophage and methods for controlling plant diseases caused by *X. fastidiosa,* wherein the bacteriophage is at least one XfaslOO-type or Xfas300-type bacteriophage selected from the group consisting of a XfaslOO phage, a Xfas300 phage, wherein the XfaslOO phage type displays certain kind of desired characteristics.

The disclosed bacteriophage is able to infect and to lysing X. *fastidiosa* bacterial cells thus preventing the disease to install and develop in plants.

Document EP0552559A2 discloses transgenic potato plants expressing the antimicrobial peptide magainin from Xenopus laevis having increased resistance to the bacteria Erwinia carotovora.

Despite all these efforts, this disease is a real threat especially because there are no chemical curative measures for the bacterium and therefore control of the disease relies only on prevention measures such as the use of resistant cultivars and cultural and hygienic practices.

Therefore, there is an urgency to find new strategies to control this disease.

The present invention proposes a method and a phytosanitary composition based on a virus peptide that overcome the disadvantages of the prior art by providing a low cost, effective and rapid and selectively strategy against Quick Decline Syndrome, in particular affecting olive-trees and olive production. A further advantage is the environmentally friend use of the resulting products that are free from toxic chemicals and does not require genetic modification of plants, especially in the case of low growing rate plants as trees.

### Summary of the invention

The present invention relates to a viral molecule comprising a virus-based vector that is able to confer protection of plants against *Xylella fastidiosa,* the pathogen that causes the Quick Decline Syndrome in olive. In another aspect, the invention relates to a method for controlling said bacteria in plants by applying a phytosanitary composition comprising a virus-based vector, carrying a peptide sequence that provides plant protection against *Xylella fastidiosa.*

Thus, in one aspect the present invention relates to a vector based on a plant virus, which is modified to carry an antimicrobial sequence in plants thereby conferring them protection against *X. fastidiosa* according to claim 1.

The resulting vector is useful to be produce phytosanitary compositions that can be used for controlling diseases in plants caused by *X. fastidiosa,* according to claim 2.

This product is of great interest for plant protection strategies, in particular concerning olive crops, since, when introduced in olive plants protects them from *X. fastidiosa* that causes Quick Decline Syndrome in olive, a disease of major concern for olive producers all over the world being responsible for extremely significant economic losses in this crop and that lacks means of control.

The possibility of producing products free from toxic chemicals is particularly important in food quality and safety, as there is an increasing tendency for consumers to demand healthier products that are more valued than products from conventional agriculture.

In another aspect, the present invention relates to a method of controlling Quick Decline Syndrome caused by *X. fastidiosa* by providing an adequate amount of a product comprising a vector as described above, according to claim 5.

This strategy is a breakthrough in controlling of this disease since it is not harmful to the environment or public health and contributes to the end of the use of the great amounts of pesticides currently used in crops to control the disease vectors.

Additionally, it does not require plant transformation, generating lower costs and effective and rapid results.

The method of the invention allows to intensify and improve the olive production, also allowing the differentiation and anticipation of the productions, increasing the competitiveness of the agri-food sector.

### General description of the invention

The present invention relates to a viral molecule comprising a virus-based vector that is able to confer protection of plants against *Xylella fastidiosa,* the pathogen that causes the Quick Decline Syndrome in olive. In another aspect, the invention relates to a method for controlling said bacteria in olive plantlets by applying a phytosanitary composition comprising a virus-based vector, carrying a peptide sequence that provides plant protection against *Xylella fastidiosa.*

The virus vector herein provided is based on *Olive mild mosaic virus* (OMMV) being the infectious transcript of OMMV cDNA clone obtained from an OMMV modified to become non-fungal transmitted. This virus mutant is further modified to cause no disease symptoms in plants and hereinafter named **OMMV_noSnoT.**

An antimicrobial sequence SEQ.ID.1 from *Xenopus laevis magainins* L. homeolog (magainins.L) having the accession number NM_001087837.1 commercially obtained and allowing the synthesis of the antibiotic magainin II is provided and is ligated with T4 DNA ligase into OMMV_noSnoT, previously digested with the restriction enzyme HPaI, that cuts in the position nt3454, 9 nt downstream of the CP and leaves blunt ends.

The resulting vector comprising OMMV_noSnoT and the antibiotic sequence SEQ. ID. No. 1, is hereinafter termed **Xylellfree vector.** Xylellfree vector is stable, it replicates and accumulates in *Nicotiana benthamiana* plants, causing no disease symptoms and is able to express the antibiotic in amounts that allow conferring protection of plants against *Xylella fastidiosa.*

### 1. Construction of a virus vector

The virus a vector of the present invention is based on *Olive mild mosaic virus* (OMMV), a virus originally isolated from olive by Cardoso et *al.* as described in Cardoso, J. M. S., Félix, M. R., Clara, M. I. E. & Oliveira, S. (2005). The complete genome sequence of a new necrovirus isolated from Olea europaea L. Arch Virol 150, 815-823.

An infectious transcript of OMMV cDNA clone obtained from OMMV type strain (Accession number HQ651834.1) is then modified to become non-fungal transmitted, through a single mutation from adenine to thymine at nt 3200 resulting in a non-synonymous change from asparagine to tyrosine at position 189 of the coat protein (CP) as described by Varanda et *al.* in Varanda, C.M.R.; Félix, M.R.; Soares, C.M.; Oliveira, S.; Clara, M.I.E. (2011). Specific amino acids of Olive mild mosaic virus coat protein are involved on transmission by Olpidium brassicae. Journal of General Virology, 92 (9): 2209-2213.

The resulting OMMV_noSnoT mutant virus is further modified to cause no disease symptoms in plants, through 3 point mutations: cytosine to guanine at nt 3359 (proline to alanine at position 242 of CP aa sequence); guanine to cytosine at nt 3365 (alanine to proline at position 244 of CP aa sequence) and guanine to thymine at nt 3368 (alanine to serine at position 245 of CP aa sequence.

Mutations were introduced using the QuikChange Site-Directed Mutagenesis kit Stratagene, in accordance to the instruction manual.

Primers designed for this specific purpose with the **SEQ.ID 2** and **SEQ.ID.3** are used according to the primer design guidelines:
**Mut** _OMMV_**Fwd:**
   **SEQ.ID.2:** 5' GCTGGGGACGGCGGAGCTGTGCCTTCCACAGCTGTGGGC 3',
   and
**Mut _OMMV_Rev:**
   **SEQ.ID.3:** 5' GCCCACAGCTGTGGAAGGCACAGCTCCGCCGTCCCCAGC;
wherein the mutations are underlined.

Mutation strand synthesis reaction was, for a final volume of 50 µL, as follows: 5 µL of 10x reaction buffer, 50 ng of plasmid DNA (OMMV in puC18), 125 ng of SEQ.ID.2 (Mut_OMMV_Fwd) and 125 ng of SEQ.ID.3 (Mut_OMMV_Rev), 1 µL of dNTP mix and 2,5 U of PfuTurbo DNA polymerase.

Cycling parameters consisted of: 1 cycle at 95 °C for 30 seconds; 18 cycles of 95 °C at for seconds, 55 °C for 1 minute and 68 °C for 6 minutes. Reaction was placed on ice for 2 minutes to cool the reaction. Digestion of amplification products was performed through Dpn I digestion to digest the parental dsDNA. 1 µL Dpn I restriction enzyme was added to the reaction and incubated at 37 °C for 1 hour. Transformation was performed using XL1-Blue Supercompetent cells following the manufacturer's instructions.

The antimicrobial sequence SEQ ID No. 1 is ligated with T4 DNA ligase into OMMV_noSnoT, previously digested with the restriction enzyme HPaI, that cuts in the position nt3454, 9 nt downstream of the CP and leaves blunt ends.

The resulting vector comprising OMMV_noSnoT plus antibiotic, is hereinafter termed **Xylellfree vector.** Xylellfree vector is stable, it replicates and accumulates in *Nicotiana benthamiana* plants, causing no disease symptoms and is able to express the antibiotic in amounts that allow conferring protection of plants against *Xylella fastidiosa.*

In short, we have obtained a virus-based vector (Xylellfree vector) for plant protection against *Xylella fastidiosa* comprising on the modified genome of *Olive mild mosaic virus* (OMMV) having the accession number HQ651834.1 with an antimicrobial sequence from *Xenopus laevis magainins* having the accession Number NM_001087837.1, which is placed downstream of the CP the OMMV full genome with:
- A mutation from adenine to thymine at nt 3200;
- A mutation from cytosine to guanine at nt 3359;
- A mutation from guanine to cytosine at nt 3365;
- A mutation from guanine to thymine at nt 3368;
- The SEQ.ID.1 introduced at nt 3454.

### 2. Phytosanitary compositions comprising Xylellfree vector

Phytosanitary compositions conferring protection of plants against Quick Decline Syndrome caused by *Xylella fastidiosa* can be produced comprising a virus-based vector as described in the previous section.

Said compositions can be presented in the form of suspension and spray that are directly applicable to plantlets to be treated.

### 3. Method for controlling Quick Decline Syndrome in olive

The virus-based vector described in the previous section, for conferring protection of plants against *Xylella fastidiosa* that causes the Olive quick decline syndrome can be produced and maintained in *Nicotiana benthamiana* plants. Macerated leaves will be subjected to viral purification as described in Varanda et al., 2011. Viral suspensions will be used to spray inoculate plants, after loading the mixture into an airbrush attached to a compressor set which will be used to spray plant leaves.

### SEQUENCE LISTING

### (i) DNA sequence listing:

SEQ.ID.1: 
TTGAAATACAACTATATTTGGAAAAGATGTTCAAAGGATTATTTATCTGTTCACTAATTG
CTGTGATCTGTGCAAATGCACTGCCACAACCAGAGGCCTCTGCGGATGAAGATATGGATG
AAAGAGAGGTCCGGGGAATTGGTAAATTTTTGCATTCAGCGGCAAATTTGGAAAAGCTT
TTGTGGGAGAGATAATGAAGTCAAAACGAGATGCAGAAGCAGTAGGACCAGAGGCCTTTG
CAGATGAAGATTTAGATGAAAGAGAGGTCCGGGGAATTGGTAAATTTTTGCACTCAGCAA
AAAAATTTGGAAAAGCTTTTGTGGGAGAGATAATGAATTCAAAACGAGATGCAGAAGCAG
TAGGACCAGAGGCCTTTGCAGATGAAGATTTAGATGAAAGAGAGGTCCGGGGAATTGGTA
AATTTTTGCACTCAGCAAAAAAATTTGGAAAAGCTTTTGTGGGAGAAATAATGAATTCAA
AACGAGATGCAGAAGCAGTAGGACCAGAGGCCTTTGCAGATGAAGATTTAGATGAAAGAG
AGGTCCGGGGAATTGGTAAATTTTTGCACTCAGCAAAAAAATTTGGAAAAGCTTTTGTGG
GAGAAATAATGAATTCAAAACGAGATGCAGAAGCAGTAGGACCAGAGGCCTTTGCAGATG
AAGATTTCGATGAAAGAGAGGTCCGGGGAATTGGTAAATTTTTGCACTCAGCAAAAAAAT
TTGGAAAAGCTTTTGTGGGAGAAATAATGAATTCAAAACGAGATGCAGAAGCAGTAGGAC
CAGAGGCCTTTGCAGATGAAGATTTAGATGAAAGAGAGGTCCGGGGAATTGGTAAATTTT
TGCACTCAGCAAAAAAATTTGGAAAAGCTTTTGTGGGAGAAATAATGAATTCAAAACGAG
ATGCAGAAGCAGTAGATGACAGGAGATGGGTTGAATAGTTTCAGCAACAGAAAAACATCA
CTGAAGAACAATGATTCCCCTGAAAATGTGTAAACGGCTGAAATGATTCTGAACATACTA
GTGCTGAATAAAAAGAAGATCCCTTAGATAACCTATACCTACAGTATATATACCTACAAA
TAAACTGAGCATCACAAT
SEQ.ID.2: GCTGGGGACGGCGGAGCTGTGCCTTCCACAGCTGTGGGC
SEQ.ID.3: GCCCACAGCTGTGGAAGGCACAGCTCCGCCGTCCCCAGC

## Claims

1. A **virus-based vector (Xylellfree vector)** for plant protection against *Xylella fastidiosa* comprising on the modified genome of *Olive mild mosaic virus* (OMMV) having the accession number HQ651834.1 with an antimicrobial sequence from *Xenopus laevis magainins* having the accession Number NM_001087837.1, which is placed downstream of the CP the OMMV full genome with:
- A mutation from adenine to thymine at nt 3200;
- A mutation from cytosine to guanine at nt 3359;
- A mutation from guanine to cytosine at nt 3365;
- A mutation from guanine to thymine at nt 3368;
- The **SEQ.ID.1** introduced at nt 3454.

2. A **phytosanitary composition** for plant protection against *Xylella fastidiosa* comprising a virus-based vector **(Xylellfree vector)** as described in claim 1.

3. A phytosanitary composition according to claim 2 comprising a suspension of the virus vector as described in claim 1.

4. A phytosanitary composition according to any of the claims 2 or 3 comprising in the form of spray to inoculate plants, after loading the mixture into an airbrush attached to a compressor set which is sprayed to the plant leaves.

5. A **method** for controlling Quick Decline Syndrome in plants comprising the administration of an adequate amount of a phytosanitary composition as described in any of the claims 2 to 4.

## Patentansprüche

1. **Virusbasiertes Vektor** (**Xylellfree-Vektor**) zum Schutz von Pflanzen gegen *Xylella fastidiosa,* der das modifizierte Genom des *Olive mild mosaic virus* (OMMV) mit der Zugangsnummer HQ651834.1 sowie eine antimikrobielle Sequenz aus *Xenopus laevis-Magaininen* mit der Zugangsnummer NM_001087837.1 umfasst, die stromabwärts des CP des vollständigen OMMV-Genoms angeordnet ist, mit:
- einer Mutation von Adenin zu Thymin bei Nt 3200;
- einer Mutation von Cytosin zu Guanin bei Nt 3359;
- einer Mutation von Guanin zu Cytosin bei Nt 3365;
- einer Mutation von Guanin zu Thymin bei Nt 3368;
- die an Nt 3454 eingefügte **SEQ.ID.1**.

2. P**hytosanitäre Zusammensetzung** zum Schutz von Pflanzen gegen *Xylella fastidiosa,* umfassend einen virusbasierten Vektor (**Xylellfree-Vektor**), wie in Anspruch 1 beschrieben.

3. Phytosanitäre Zusammensetzung gemäß Anspruch 2, umfassend eine Suspension des in Anspruch 1 beschriebenen Virusvektors.

4. Phytosanitäre Zusammensetzung gemäß einem der Ansprüche 2 oder 3, umfassend in Form eines Sprühmittels zur Inokulation von Pflanzen, nachdem die Mischung in eine an eine Kompressoranlage angeschlossene Airbrush-Pistole eingefüllt wurde, mit der die Pflanzenblätter gesprüht werden.

5. **Verfahren** zur Bekämpfung des Quick-Decline-Syndroms bei Pflanzen, umfassend die Verabreichung einer ausreichenden Menge einer phytosanitäre Zusammensetzung, wie in einem der Ansprüche 2 bis 4 beschrieben.

## Revendications

1. **Vecteur à base de virus** (**vecteur Xylellfree**) destiné à la protection des plantes contre *Xylella fastidiosa*, comprenant, dans le génome modifié de l'*Olive mild mosaic virus* (OMMV) ayant le numéro d'accession HQ651834.1, une séquence antimicrobienne provenant des magainines de *Xenopus laevis* ayant le numéro d'accession NM_001087837.1, ladite séquence étant placée en aval de la CP du génome complet de l'OMMV avec :
- une mutation de l'adénine en thymine au nt 3200 ;
- une mutation de la cytosine en guanine au nt 3359 ;
- une mutation de la guanine en cytosine au nt 3365 ;
- une mutation de la guanine en thymine au nt 3368 ;
- la **SEQ. ID. 1** introduite au nt 3454.

2. C**omposition phytosanitaire** destinée à la protection des plantes contre *Xylella fastidiosa*, comprenant un vecteur à base de virus (**vecteur Xylellfree**) tel que décrit dans la revendication 1.

3. Composition phytosanitaire selon la revendication 2, comprenant une suspension du vecteur à base de virus tel que décrit dans la revendication 1.

4. Composition phytosanitaire selon l'une quelconque des revendications 2 ou 3, se présentant sous la forme d'un spray destiné à inoculer des plantes, après chargement du mélange dans un aérographe relié à un compresseur, ledit mélange étant pulvérisé sur les feuilles des plantes.

5. **Procédé** de lutte contre le syndrome du déclin rapide (« Quick Decline Syndrome ») des plantes, comprenant l'administration d'une quantité adéquate d'une composition phytosanitaire telle que décrite dans l'une quelconque des revendications 2 à 4.
